**Europäisches Patentamt**

**European Patent Office**

**Office européen des brevets**

(19)

(11) Veröffentlichungsnummer : **0 046 195**
**B1**

(12) **EUROPÄISCHE PATENTSCHRIFT**

(45) Veröffentlichungstag der Patentschrift :
08.02.84

(51) Int. Cl.³ : **C 07 D251/62, C 07 C126/08**

(21) Anmeldenummer : **81105452.7**

(22) Anmeldetag : **13.07.81**

(54) **Verfahren und Vorrichtung zur Abtrennung von flüssigem Harnstoff aus den Abgasen der Melaminsynthese.**

(30) Priorität : **18.08.80 DE 3031124**

(43) Veröffentlichungstag der Anmeldung :
**24.02.82 Patentblatt 82/08**

(45) Bekanntmachung des Hinweises auf die Patenterteilung : **08.02.84 Patentblatt 84/06**

(84) Benannte Vertragsstaaten :
**AT BE DE FR GB IT NL**

(56) Entgegenhaltungen :
**DE-A- 1 545 656**
**DE-A- 2 440 315**

(73) Patentinhaber : **BASF Aktiengesellschaft**
**Carl-Bosch-Strasse 38**
**D-6700 Ludwigshafen (DE)**

(72) Erfinder : **Borho, Klaus, Dr.**
**Dahlienstrasse 16**
**D-6704 Mutterstadt (DE)**
Erfinder : **Fromm, Dieter, Dr.**
**Auf der Halde 7**
**D-6718 Gruenstadt (DE)**
Erfinder : **Schier, Ernst-Juergen, Dr.**
**Graefenthalstrasse 14**
**D-6719 Altleiningen (DE)**
Erfinder : **Schneehage, Hans Henning, Dr.**
**Plauser Strasse 10**
**D-6719 Weisenheim (DE)**
Erfinder : **Widmann, Alfred, Dr.**
**Gartenstrasse 18**
**D-6704 Mutterstadt (DE)**

Verfahren und Vorrichtung zur Abtrennung von flüssigem Harnstoff aus den Abgasen der Melaminsynthese

Die vorliegende Erfindung betrifft ein Verfahren zur Abtrennung von flüssigem Harnstoff bzw. Gemischen aus flüssigem Harnstoff und dessen thermischen Zersetzungsprodukten aus dem bei der Synthese von Melamin anfallenden Abgasgemisch.

Für die Synthese von Melamin werden Harnstoff und/oder seine thermischen Zersetzungsprodukte in Gegenwart von Katalysatoren und von zugesetztem Ammoniak auf Temperaturen von 350-400 °C erhitzt. Zur Aufarbeitung der Melamindampf enthaltenden Synthesegase werden diese einer fraktionierten Kondensation unterworfen, d. h. sie werden auf eine Temperatur von 170-250 °C abgekühlt, bei der das Melamin selektiv und nahezu vollständig aus dem Gasstrom auskristallisiert und abgeschieden wird. Das nach der Abtrennung des Melamins verbleibende Gasgemisch, das im wesentlichen aus Ammoniak und Kohlendioxid besteht, enthält aber noch tensionsmäßig Melamin und den bei der Synthese nicht umgesetzten Harnstoff in Form seines thermischen Spaltproduktes Isocyansäure.

Zur weiteren Kühlung der Gase und zur Abtrennung dieser Verunreinigungen ist es aus der DE-PS 12 04 679 bekannt, das Gas mit einer Schmelze von Harnstoff oder einer solchen eines Gemisches aus Harnstoff und dessen thermischen Zersetzungsprodukten zu waschen. Beim Waschen mit geschmolzenem Harnstoff werden vom Gasstrom zwangsläufig kleine Harnstofftröpfchen mitgerissen, die in nachgeschalteten Tropfenabscheidern, z. B. Füllkörperkolonnen, Lamellarabscheidern oder Zyklonen abgeschieden werden müssen. Die Abscheideflächen dieser Apparate verkrusten bei längerer Betriebsdauer durch in Harnstoffschmelze unlösliche Zersetzungsprodukte des Harnstoffs, wie z. B. Cyanursäure, Melamincyanurat und dgl.

Diese Verkrustung tritt besonders an Apparateflächen auf, an denen der flüssige Harnstoff längere Zeit den erhöhten Temperaturen ausgesetzt ist. Im Extremfall kann ein an einer Abscheidefläche haftender Tropfen vollständig in seine Zersetzungsprodukte überführt werden.

Es ist weiterhin bekannt (DE-PS 16 70 216), zur Beseitigung solcher Verkrustungen die Tropfenabscheider periodisch mit Harnstoffschmelze zu fluten. Um einen kontinuierlichen Betrieb zu gewährleisten, müssen dem Harnstoffwascher mehrere dieser Tropfenabscheider nachgeschaltet werden, die wechselweise in Betrieb sind oder durch Fluten mit Harnstoffschmelze gereinigt werden.

In der DE-AS 24 40 315 wird vorgeschlagen, diesen Nachteil dadurch zu umgehen, daß man die Gase, die die mitgerissenen Flüssigkeitströpfchen enthalten, durch einen Separator (Zyklonseparator, Aufprallseparator mit Ablenkplatten oder Lamellenseparator) leitet, um die Flüssigkeitströpfchen durch Kollision mit der inneren Oberfläche des Separators abzutrennen, wobei man diese Oberflächen mit einem abwärts strömenden Film aus geschmolzenem Harnstoff, einer geschmolzenen Mischung aus Harnstoff und den thermischen Zersetzungsprodukten des Harnstoffs und/oder geschmolzenen Mischungen davon mit Melamin versieht. Wenn zwar durch diese Maßnahme die Betriebsdauer eines Separators gesteigert werden kann, so bedeutet doch die Erzeugung eines geschlossenen Flüssigkeitsfilms einen erheblichen apparativen und verfahrenstechnischen Aufwand, abgesehen davon, daß auch mit diesen Maßnahmen keine befriedigenden Betriebszeiten erreicht werden können.

Bei Lamellenseparatoren kann man zwar durch Bedüsen mit Flüssigkeit auf den dem Gaseintritt zugewandten Abscheideflächen einen zusammenhängenden Flüssigkeitsfilm erzeugen, der eine Verkrustung dieser Abscheideflächen verhindert ; um die Abscheideleistung des Separators zu erhalten, ist es jedoch unbedingt erforderlich, die auf der Gasaustrittsseite liegenden Abscheideflächen mit möglichst wenig Flüssigkeit zu beaufschlagen. An diesen hinteren Abscheideflächen bilden sich deshalb stetig wachsende Krusten, welche die Abscheidefunktion beeinträchtigen, dadurch auch zur Krustenbildung in den nachfolgenden Leitungen führen und deshalb in regelmäßigen Abständen zum Abstellen und Reinigen zwingen, obwohl der Abscheider mit einem Schmelzefilm versehen worden ist.

Mit Zyklonseparatoren, die ebenfalls in der genannten DE-AS erwähnt werden, lassen sich bezüglich der Abscheidung von Harnstoffschmelzetröpfchen in noch geringerem Maße befriedigende Ergebnisse erzielen. Wie aus der Literatur bekannt ist (vgl. Chemie-Ingenieur-Technik 25, (1953), Seiten 328-330), müssen bei der Abscheidung von Flüssigkeiten aus Gasen in Zyklonen besondere Maßnahmen ergriffen werden um ein Mitreißen der Flüssigkeit durch das den Abscheider verlassende Gas zu verhindern. Dieses Mitreißen von Flüssigkeit wird dadurch verursacht, daß die an die Außenwandung des Zyklons geschleuderte Flüssigkeit unter dem Einfluß der zwischen Zentrum und Peripherie des Wirbelfeldes bestehenden Druckdifferenz am Deckel entlang zu dem in der Mitte des Zyklondeckels angeordneten und als Gasauslaß dienenden Tauchrohres kriecht, an diesem nach unten läuft und dann dort von der aufwärts gerichteten Gasströmung erfaßt und mitgerissen wird.

Bei normalen Flüssigkeiten kann dieses Kriechen durch konzentrische, um das Auslaßrohr angeordnete Ringe reduziert werden. Zur Abscheidung der auch diese Einbauten überwindenden geringen Flüssigkeitsmenge wird in der oben bereits erwähnten Literaturstelle empfohlen, hinter dem Gasauslaßrohr weitere konstruktive Maßnahmen zur Ableitung dieser an der Innenwand des Tauchrohres entlang kriechenden Flüssigkeit zu treffen.

Bei der Abscheidung von Harnstoffschmelzetröpfchen aus den Abgasen der Melaminsynthese scheiden derartige Maßnahmen aus, da sich gerade der an diesen Stellen ansammelnde Harnstoff bevorzugt thermisch zersetzt und zur Bildung von Verkrustungen führt. Diese Schwierigkeiten lassen sich auch nicht dadurch beheben, daß man die Wandungen des Zyklons zusätzlich mit einem abwärts strömenden Film aus geschmolzenem Harnstoff versieht.

Der vorliegenden Erfindung lag daher die Aufgabe zugrunde, ein Verfahren zur Abtrennung von flüssigem Harnstoff oder Gemischen von flüssigem Harnstoff und dessen thermischen Zersetzungsprodukten aus den bei der katalytischen Umwandlung von Harnstoff bei erhöhten Temperaturen anfallenden Abgasen der Melaminsynthese, die durch fraktionierte Kondensation von Melamin befreit worden sind un anschließend mit geschmolzenem Harnstoff oder Mischungen aus geschmolzenem Harnstoff und dessen thermischen Zersetzungsprodukten gewaschen worden sind, bereitzustellen, das ohne großen apparativen und verfahrenstechnischen Aufwand durchgeführt werden kann, das ferner eine wirksame Trennung des Gases von der flüssigen Phase gewährleistet und durch das gleichzeitig die Bildung von Verkrustungen im Abscheidesystem und in den dem Abscheidesystem nachgeschalteten Leitungen vermieden wird.

Es wurde gefunden, daß diese Aufgabe dadurch gelöst werden kann, daß das aus der Waschzone kommende Gas-Flüssigkeitsgemisch, das pro kg Gas 2 bis 10 kg der Schmelze enthält, mit einer Geschwindigkeit von 8 bis 30 m/s tangential in den oberen Teil einer zylindrischen Abscheidezone eingeführt wird, wobei durch die dem Gas-Flüssigkeitsgemisch erteilte Rotation die beiden Phasen voneinander getrennt und beide Phasen gleichsinnig von oben nach unten durch die Abscheidezone bewegt werden und Gas und abgeschiedene Flüssigkeit am unteren Ende der Abscheidezone getrennt voneinander abgezogen werden, wobei die mittlere Verweilzeit des Gasgemisches in der Abscheidezone 0,5 bis 5 Sekunden beträgt.

Üblicherweise werden die von Melamin befreiten Gase mit 5 bis 10 kg Harnstoffschmelze oder Mischungen aus geschmolzenem Harnstoff und dessen thermischen Zersetzungsprodukten je kg Gas gewaschen. Im Gegensatz zu den bekannten Verfahren werden also bei dem erfindungsgemäßen Verfahren nicht nur die vom Gas mitgerissenen Schmelzetröpfchen, sondern besonders vorteilhaft die gesamte oder aber zumindest ein erheblicher Teil der Waschflüssigkeit zusammen mit dem Gas in die Abscheidezone eingeführt.

Überraschenderweise gelingt es bei dem erfindungsgemäßen Verfahren, die Wände der Abscheidezone und der nachgeschalteten Leitungen von Verkrustungen durch thermische Zersetzungsprodukte des Harnstoffs wie Cyanursäure oder Melamincyanurat frei zu halten und damit eine Verringerung der Trennleistung zu vermeiden, ohne daß man die Separatoroberflächen mit einem abwärtsströmenden Flüssigkeitsfilm versieht und ohne daß die Abscheidezone durch periodisches Fluten mit Harnstoffschmelze oder geschmolzenen Gemischen aus Harnstoff und dessen thermischen Zersetzungsprodukten gereinigt wird.

Ein weiteres Merkmal des erfindungsgemäßen Verfahrens ist die gleichsinnige Führung von Gas und der durch die Zentrifugalkräfte abgeschiedenen Flüssigkeit durch die Abscheidezone, wodurch die Verweilzeit der Schmelze und damit die Bildung von Zersetzungsprodukten verringert wird.

Weiterhin ist die Einhaltung der oben angegebenen Geschwindigkeitsgrenzen am Eintritt in die Abscheidezone von Bedeutung, wobei eine ausreichende Abscheidung erst bei Geschwindigkeiten $\geqq 8$ m/s gewährleistet ist, während andererseits Geschwindigkeiten von 30 m/s nicht überschritten werden sollen, da sonst die an der Wandung abgeschiedenen Flüssigkeitstropfen wieder abgelöst und von dem die Abscheidezone verlassenden Gasstrom mitgerissen werden, was zu Verkrustungen und schließlich zu Verstopfungen der dem Abscheidesystem nachgeschalteten Leitungen und Apparateteile führen würde.

Die Einhaltung einer Mindestverweilzeit von 0,5 Sekunden ist für eine vollständige Abscheidung der im Gas befindlichen Tröpfchen von erheblicher Bedeutung. Theoretisch wird zwar mit zunehmender Verweilzeit das Abscheideergebnis immer besser, man wird jedoch aus praktischen Gründen und um Schmelzezersetzungen vorzubeugen, Verweilzeiten von 5 Sekunden nicht überschreiten.

Das erfindungsgemäße Verfahren sei im folgenden anhand der schematischen Figur näher veranschaulicht:

Die vom Melamin befreiten Abgase, die noch unumgesetzten Harnstoff und tensionsmäßig Melamin enthalten, treten durch Leitung (1) in den Wäscher (2) ein. Durch Leitung (3) wird als Waschflüssigkeit z. B. Harnstoffschmelze eingeführt. Am unteren Ende des Waschers werden Gas und Schmelze gemeinsam abgezogen und durch den Einlaß (4) tangential in den zylindrischen Abscheider (5) eingeführt.

Die Schmelze wird durch die ihr erteilte Rotation gegen die Wandung geschleudert und bewegt sich entlang der Wandung spiralförmig nach unten. Die sich ebenfalls nach unten bewegenden Gase verlassen die Abscheidezone durch das in den Boden der Abscheidezone hineinkragende Tauchrohr (6), während die Flüssigkeit sich am Boden ansammelt und durch das Rohr (7) abgezogen wird. In dem unteren Teil des Abscheiders beginnend mit dem oberen Ende des Tauchrohres bis zum Boden der Abscheidezone, bildet sich ein Beruhigungsraum aus, wodurch ein Mitreißen von Flüssigkeit durch den Gasstrom mit Sicherheit vermieden wird. Auch können keine kriechenden Flüssigkeitsfilme zu der oberen Öffnung des Tauchrohrs gelangen, da sie hierzu die Schwerkraft überwinden müßten. Durch den in diesem Bereich scharfen Übergang von Flüssigkeit und trockener Wand des Tauchrohres ist eine Entstehung von Krusten nicht möglich. Wie Versuche an einem durchsichtigen Modellapparat, der mit einem Gemisch aus Luft und entspanntem Wasser betrieben worden ist, gezeigt haben, bilden sich bei Einhaltung der erfindungsgemäßen Maßnahmen entlang der Wandung nach unten wandernde spiralförmige Flüssigkeitssträhnen aus, die ihre Lage statistisch verändern. Überraschenderweise wird hierdurch beim Betrieb mit einem Harnstoffschmelze enthaltenden Abgas die Ausbildung von Verkrustungen wirksam vermieden.

Die Vorteile des erfindungsgemäßen Verfahrens seien durch die folgenden Beispiele näher erläutert :

Beispiel

A. 19 000 kg/h eines von Melamin weitgehend bereits befreiten Gases werden mit 130 000 kg/h Harnstoffschmelze, die Melamin und thermische Zersetzungsprodukte des Harnstoffs enthält, gewaschen und anschließend mit einer Geschwindigkeit von 14 m/s tangential in einen Zyklon herkömmlicher Bauart mit einem Längezu Durchmesser-Verhältnis von 3 : 1 eingeleitet. Das abgetrennte Gas entweicht nach oben über ein Tauchrohr, die abgeschiedene Harnstoffschmelze wird unten abgezogen. Aus einem dem Zyklon nachgeschalteten Schwerkraftabscheider lassen sich bereits nach 14 Tagen stündlich 20 kg von im Zyklon nicht abgeschiedener Harnstoffschmelze abziehen. Nach 20 Tagen erhöht sich diese Menge auf 40 kg/h. Die Abscheideleistung des Zyklons läßt sich durch Veränderung des Gasdurchsatzes nicht verbessern.

Bereits nach 14 Tagen zeigen sich im Tauchrohr und in den nachfolgenden Gasleitungen starke Ablagerungen von Harnstoffzersetzungsprodukten.

B. Das in A) beschriebene, aus der Harnstoffwäsche einer Melaminanlage kommende Gas-Flüssigkeitsgemisch wird mit einer Geschwindigkeit von 24 m/s tangential in eine zylindrische Abscheidezone geleitet und durchströmt diese mit einer Axialgeschwindigkeit von 4 m/s von oben nach unten. Nach einer Verweilzeit von 2 Sekunden verläßt das vom Harnstoff befreite Gasgemisch die Abscheidezone am unteren Ende durch ein zentrales Rohr. Die abgetrennte Harnstoffschmelze wird ebenfalls unten abgezogen. Aus dem der Abscheidezone nachgeschalteten Schwerkraftabscheider kann auch nach 8-monatiger Betriebszeit kein flüssiger Harnstoff abgezogen werden. An den Wänden des Gasabgangsrohres und in den nachfolgenden Leitungen bilden sich während dieser Zeit keine störenden Ablagerungen von Harnstoffzersetzungsprodukten. Die Abscheideleistung der Abscheidezone bleibt auch bei betriebsbedingten Schwankungen der Gasmenge von 14 000-

22 000 kg/h unverändert.

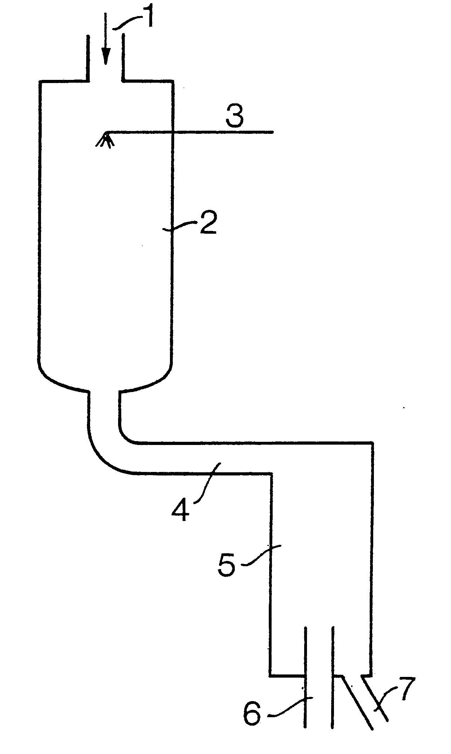

## Ansprüche

1. Verfahren zur Abtrennung von flüssigem Harnstoff oder Gemischen von flüssigem Harnstoff und dessen thermischen Zersetzungsprodukten aus den bei der katalytischen Umwandlung von Harnstoff bei erhöhten Temperaturen anfallenden Abgasen der Melaminsynthese, die durch fraktionierte Kondensation von Melamin befreit worden sind und anschließend mit geschmolzenem Harnstoff oder Mischungen aus geschmolzenem Harnstoff und dessen thermischen Zersetzungsprodukten gewaschen worden sind, dadurch gekennzeichnet, daß das aus der Waschzone kommende Gas-Flüssigkeitsgemisch, das pro kg Gas 2 bis 10 kg der Schmelze enthält, mit einer Geschwindigkeit von 8 bis 30 m/s tangential in den oberen Teil einer zylindrischen Abscheidezone eingeführt wird, wobei durch die dem Gas-Flüssigkeitsgemisch erteilte Rotation die beiden Phasen voneinander getrennt und beide Phasen gleichsinnig von oben nach unten durch die Abscheidezone bewegt werden und Gas und abgeschiedene Flüssigkeit am unteren Ende der Abscheidezone getrennt voneinander abgezogen werden, wobei die mittlere Verweilzeit des Gasgemisches in der Abscheidezone 0,5 bis 5 Sekunden beträgt.

2. Vorrichtung zur Durchführung des Verfahrens nach Anspruch 1, gekennzeichnet durch ein zylindrisches Abscheiderohr (5) mit einem an dessen oberem Ende tangential einmündenden Einlaßrohr (4) für die Einführung des mit Schmelze beladenen Abgases, einem vom Boden des Abscheiderohres in den Innenraum hineinkragenden Tauchrohr (6) für den Abzug des Gases und einem am Boden angeordneten Rohr (7) für den Abzug der Schmelze.

## Claims

1. A process for isolating liquid urea or a mixture of liquid urea and its thermal decomposition products from the off-gases of the synthesis of melamine, which are formed during the catalytic conversion of urea at an elevated temperature, and have been freed from melamine by fractional condensation and subsequently been washed with molten urea or a mixture of molten urea and its thermal decomposition products, wherein the gas-liquid mixture coming from the wash zone and containing 2-10 kg of the melt per kg of gas is passed tangentially, at a velocity of 8-30 m/sec, into the upper part of a cylindrical separation zone, where, due to the rotation imparted to the gas-liquid mixture, the two phases are separated from one another and both are moved downward, in the same direction, through the separation zone, and gas and separated-out liquid are taken off separately from one another at the bottom end of the separation zone, the mean residence time of the gas mixture in the separation zone being from 0.5 to 5 seconds.

2. An apparatus for carrying out the process as claimed in claim 1, comprising a cylindrical separating tube (5) with an inlet tube (4) for the introduction of the off-gas laden with melt, which inlet tube enters the upper end of the separating tube tangentially ; a dip tube (6) for the withdrawal of the gas, said tube projecting from the bottom of the separating tube into the interior thereof ; and a tube (7), located in the bottom of the separating tube, for the withdrawal of the melt.

**Revendications**

1. Procédé pour la séparation de l'urée liquide ou de mélanges d'urée liquide et de produits de dégradation thermique de celle-ci des gaz résiduaires provenant de la synthèse de la mélamine par transformation catalysée de l'urée à des températures accrues, desquels on a éliminé la mélamine par une condensation fractionnée et qui ont ensuite été lavés avec de l'urée fondue ou un mélange d'urée fondue et de produits de la dégradation thermique de celle-ci, caractérisé en ce que le mélange liquide-gaz, quittant la zone de lavage, qui contient par kg de gaz entre 2 et 10 kg de produits fondus, est introduit tangentiellement, avec une vitesse de 8 à 30 m/s, dans la partie supérieure d'une zone de séparation cylindrique, dans laquelle la rotation imprimée au mélange liquide-gaz provoque la séparation des deux phases, qui traversent la zone de séparation de haut en bas en courants parallèles, le gaz et le liquide séparé étant soutirés séparément à l'extrémité inférieure de la zone de séparation après une durée de séjour moyenne du mélange gazeux de 0,5 à 5 secondes dans la zone de séparation.

2. Dispositif pour la mise en œuvre du procédé selon la revendication 1, caractérisé en ce qu'il comprend un tube de séparation cylindrique (5), un tuyau d'entrée (4) pour l'introduction du gaz résiduaire chargé de produits fondus, débouchant tangentiellement dans la partie supérieure du tube de séparation, un tube plongeur (6) pour l'évacuation du gaz, faisant saillie du fond du tube de séparation vers l'intérieur, et un tuyau (7) pour l'évacuation des produits fondus, partant du fond du tube de séparation.